# EUROPEAN PATENT APPLICATION

(11) **EP 1 884 211 A2**
(43) Date of publication of application: **06.02.2008**
(21) Application number: 07253047.0
(22) Date of filing: 02.08.2007
(51) Int. Cl.: A61B 17/34, A61B 17/00

(54) **Optical device for needle placement into a joint**

(30) Priority: 02.08.2006 US 497238
(71) Applicant: Zuckerman, Stephen D., Beverly Hills CA 90210 (US)
(72) Inventor: Zuckerman, Stephen D., Beverly Hills CA 90210 (US)
(74) Representative: Calderbank, Thomas Roger

(57) **Abstract**

An optical device is provided which assists in accurate placement of a needle (18) into a human or animal diarthrodial joint. The device includes a handpiece (10) which mounts a needle assembly including an optical guide (25). The optical guide, which is incorporated into the lumen of the needle, transmits light (32) from the needle tip into the joint area and receives the scattered light that is returned. The handpiece is manipulated by the user to guide the needle during placement. The returned light is processed to determine whether the needle is placed in the joint itself rather than in a location adjacent to the joint and corresponding output is produced to aid the user in effecting proper needle placement. Such placement assists in the injection of fluid into or the removal of fluid from the joint.

## Description

The present invention relates to the placement of needles into the joints of humans or animals for medical diagnosis or therapy.

Although the invention is certainly not limited to injection of knee joints, this is one notable application of the invention. The importance of knee joint injection is growing. The injection of long-acting steroid preparations continues to be a mainstay of conservative management for osteoarthritis. The injection of hyaluronic acid preparations has increased, and these preparations now represent an important therapy for osteoarthritis.

Historically, knee joint injections have been performed in the specialty setting, but there is a growing need for primary care providers to inject the knee joint routinely. Many patients with osteoarthritis of the knee are managed by primary care providers until they are candidates for joint replacement. Increasingly, specialists such as orthopedists, rheumatologists, and interventional musculoskeletal radiologists see patients in the later stages of disease.

Most knee joint injections are performed blindly, i.e., without the aid of any assisting device or imaging technology for needle placement. One method involves air insufflation technique to elicit crepitus for blind needle guidance (see Glattes RC, Spindler KP, Blanchard GM, Rohmiller MT, McCarty EC, Block J., "A simple, accurate method to confirm placement of intra-articular knee injection." Am J Sports Med. 2004 Jun; 32(4):1029-31).

Many primary care providers feel uncomfortable injecting the knee joint blindly, since they have not had the opportunity to practice this procedure in volume. Further, blind knee joint injection can be performed incorrectly even by experienced specialists (see Jackson DW, Evans NA, Thomas BM., "Accuracy of needle placement into the intra-articular space of the knee." J Bone Joint Surg Am. 2002 Sep; 84-A(9):1522-7). A missed injection can result in depositing drugs into the soft tissues surrounding the knee, such as fat, muscle, or anterior fatpad. Inaccurate injection can deprive a patient of needed therapy, cause complications, and decrease the apparent clinical effect of scientifically proven therapies.

X-ray fluoroscopy is the current standard for the guidance of needle placement for injection. Numerous academic articles have described multiple aspects of fluoroscopically guided needle placement in various joints. Ultrasonography has been used for image-guided injection of joints and bursa (see Naredo E, Cabero F, Palop MJ, Callado P, Cruz A, Crespo M., "Ultrasonographic findings in knee osteoarthritis: a comparative study with clinical and radiographic assessment." Osteoarthritis Cartilage. 2005 Jul; 13(7):568-74). However, this procedure has numerous disadvantages, including radiation exposure.

Arthroscopy, i.e., the use of optical devices to visualize and treat the knee and other joints, is a routine surgical procedure. Numerous patents discuss methods and devices relating to arthroscopic cannulas, trocars, obturators, guides, arthroscopes and related equipment. For example, a small diameter cannular, trocar, and arthroscope system is described in U.S. Patent Number 6,695,772 to Bon et al.. This system is similar to a very large needle that is to be used in an office setting. Similarly to the needle placement techniques discussed above, arthroscopy systems rely on blind placement of the initial instruments by an interventionalist with extensive manual skills.

In accordance with the invention, a device and method are provided which, among other applications, aid in the accurate injections of the knee, in a clinic or similar setting, and which thus are of benefit to both patients and primary care providers. It will be appreciated that although the injection of the knee joint is an important application, the device and method can be used in other applications involving the placement of a needle into a patient including the injection or removal of fluid from any diarthrodial joint, such as the hip, ankle, shoulder, elbow or wrist.

In accordance with one aspect of the invention, there is provided a method for positioning a needle within a patient, said method comprising :
providing a device including a needle including a needle tip and a lumen, and an optical guide disposed in the lumen of the needle and extending to the needle tip;
positioning the needle within a body substance of a patient by piercing the skin and soft tissue of the patient;
causing light to be transmitted through the optical guide so as to be emitted from the needle tip into the patient;
using the optical guide to receive light returned from the body substance of the patient in which the needle tip is positioned; and
processing the returned light to provide a determination of the body substance in which the needle tip is positioned.

Preferably, parameters relating to both the transmitted light and the returned light are processed in providing said determination.

In a preferred implementation, the transmitted and returned light are compared with respect to relative intensity. Advantageously, properties of different body substances are used for said determination. Beneficially, the determination includes discriminating between body substances selected from the group consisting of connective tissue, muscle, fat, synovial tissue, synovial fluid, and intra-articular connective tissue.

Preferably, the method further comprises displaying an indication of the probability that the needle tip is positioned in an intra-articular space within the patient. Advantageously, the method further comprises repositioning the needle, as needed, until the indication displayed represents an acceptable probability that the needle tip is positioned in the intra-articular space.

In one preferred embodiment, the transmitted light is produced by a light source, the returned light is converted into an electrical signal, and the electrical signal and an electrical signal from a power supply for the light source are processed to provide an input in a parameter estimation process that provides said determination.

Preferably, the device comprises a handpiece and at least part of said processing takes place within the handpiece.

According to a further aspect of the invention, there is provided a device for assisting in positioning of a needle within a patient, the device comprising:
a handpiece for manipulation by a user;
a light source;
a needle assembly mounted on the handpiece, said needle assembly comprising a needle including a needle tip and a central lumen, and an optical guide, disposed in said lumen, for, in use with the needle inserted in the patient, transmitting light from said light source through said lumen so as to be emitted from the needle tip and receiving the light emitted from the needle tip that is returned to the needle tip from a location within the patient; and
processing means for processing the returned light to provide a determination of the location within the patient at which the needle tip is positioned.

In one preferred embodiment, the optical guide comprises at least first and second fiber optics supported in the lumen.

Preferably, the processing means uses parameters related to both the transmitted and the returned light in providing said determination. In one advantageous implementation, the processing means compares the transmitted and returned light with respect to relative intensity. Preferably, the processing means uses properties of different body substances in said determination. Advantageously, the determination by the processing means includes discriminating between body substances selected from the group consisting of connective tissue, muscle, fat, synovial tissue, synovial fluid, and intra-articular connective tissue.

Preferably, the device further comprises readout means for displaying an indication of the probability that the needle tip is positioned in an intra-articular space within the patient.

Preferably, the device further comprises a parameter estimation module, and a power supply for producing an electrical output for powering the light source, and the processing means includes means for converting the returned light into an electrical signal, and means for comparing the electrical signal and said electrical output from said power supply for the light source to provide an input to said parameter estimation module.

Advantageously, at least part of the processing by the processing means takes place within the handpiece.

According to yet another aspect of the invention, there is provided a device for assisting in positioning of a needle within a patient, the device comprising:
a handpiece for manipulation by a user;
a light source;
a needle assembly mounted on the handpiece, said needle assembly comprising a needle including a needle tip and a central lumen and an optical guide disposed in said lumen and including at least a first optical fiber for, in use with the needle inserted in the patient, transmitting light from said light source through said lumen so as to be emitted from the needle tip and at least a second optical fiber for, in use with the needle inserted into the patient, receiving the light emitted from the needle tip that is returned to the needle tip from a location within the patient;
processing means for processing the returned light to provide a determination of the location within the patient at which the needle tip is positioned; and
a readout, connected to said processing means, for indicating to a user, based on said determination, a probability of the needle tip being located at a predetermined location in the patient.

Preferably, the readout comprises at least two different light outputs indicating at least two different probabilities that the needle tip is located at said predetermined location.

Preferably, the predetermined location is within an intra-articular space within the patient.

Further features and advantages of the present invention will be set forth in, or apparent from, the detailed description of preferred embodiments thereof which follows.

IN THE DRAWINGS:
Figure 1 is a front perspective view of a handpiece constructed in accordance with one preferred embodiment of the invention;
Figure 2 is a fragmentary side elevational view of the needle tip of Figure 1;
Figure 3 is a cross-sectional view taken generally along line III-III of Figure 2;
Figure 4 is an end elevational view of the needle tip of Figure 2;
Figure 5 is a cross-sectional view of the handpiece of Figure 1 with the needle assembly removed;
Figure 6 is a perspective view of a control unit for the handpiece of Figure 1, including a fragmentary portion of that handpiece;
Figure 7 is a block diagram of one preferred embodiment of the overall system;
Figure 8 is a block diagram of another preferred embodiment of the overall system;
Figure 9 is a block diagram of a preferred embodiment of the parameter estimation system of Figures 7 and 8;
Figure 10 is a block diagram showing further details of one preferred embodiment of the parameter estimation system which uses a lookup table; and
Figure 11 is a block diagram of one preferred embodiment of implementing the lookup table of Figure 10.

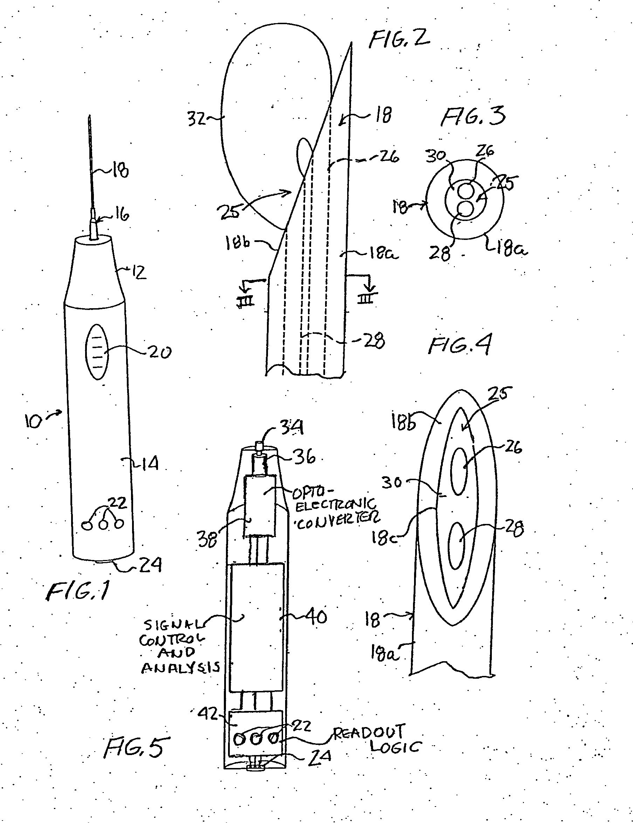

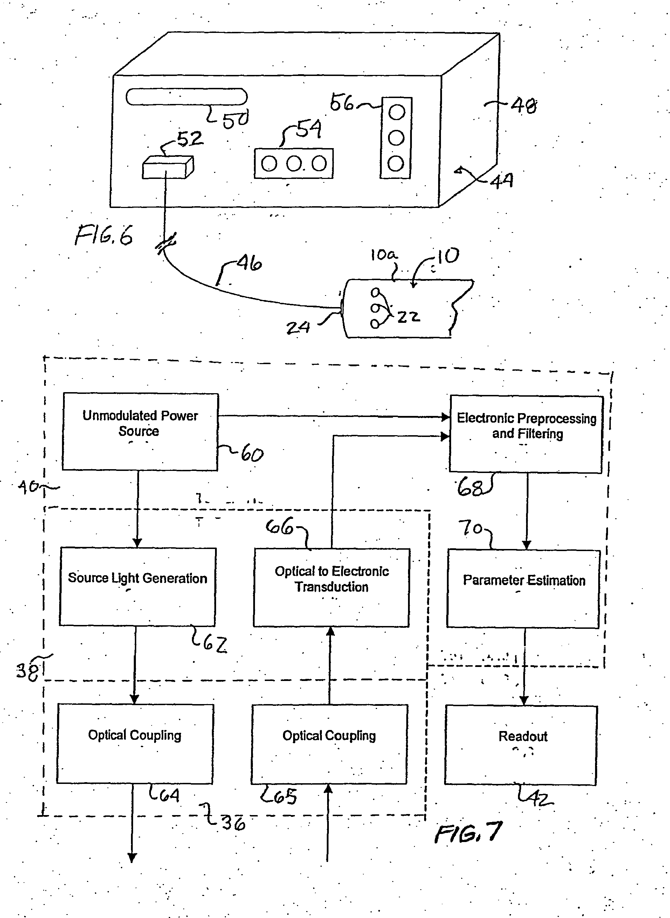

Referring to Figure 1, there is shown a perspective view of one preferred embodiment of a handpiece 10 including a tapered nose portion 12 and a body portion 14. Nose portion 12 supports a mounting element or needle base 16, preferably made of plastic, at the distal end thereof which mounts a needle 18 that is described in more detail below. Needle base 16, which is preferably made of plastic is, in a preferred embodiment, permanently affixed to the shaft of needle 18 and detachably mounted on the distal end or nose portion of body portion 14 as described in more detail below. Body portion 14 includes a control button 20 and a series of readout devices 22. The proximate end of body portion 14 includes an electrical connector or communication coupling 24 for power and/or control logic input.

It will, of course, be understood that handpiece 10 may take other forms. In addition, needle 18 and handpiece 10 may be connected by a friction coupling, a luer lock coupling, or another suitable needle-to-syringe coupling, and handpiece 10 can be fixed to needle 18 or can be removable as described above.

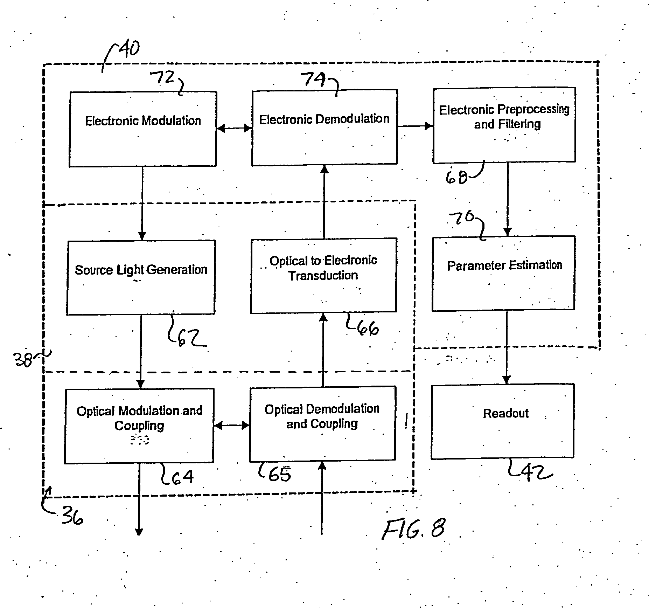

Referring to Figures 2 to 4, the needle 18 of Figure 1 is shown in more detail. Needle 18 includes a shaft 18a that terminates in a slant distal end 18b and an internal bore or lumen 18c. An optical guide or light guide 25 comprising first and second fiber optic elements 26 and 28 mounted within a support member 30 disposed in lumen 18c. Member 30, which is preferably made of a suitable polymer resin, supports the elements 26 and 28 in spaced relation in lumen 18c.

In alternative embodiments, the optical guide 25 can be fixed in, or removable from, the lumen 18c of needle 18.

In an advantageous implementation, optical fibers 26 and 28 are multi-mode fibers with a variable core width but with a 125 micrometer cladding. In this implementation, needle 18 is a standard commercial needle and the optical guide 25, comprising the bundled optical fibers 26 and 28 with the encapsulating polymer resin member 30, forms a trocar assembly that fits down the lumen 18c of needle 18.

In alternative embodiments, needle 18 may be a standard medical needle with a gauge of from 7 to 30 corresponding to an inner diameter of from 3.81 mm to 0.15 mm and an outer diameter of from 4.57 to 0.31 mm or needle 18 can be a custom, specially designed needle or an arthroscopic cannula.

In alternative implementations, optical guide 25 may comprise a single mode fiber optic, a plurality of optical fibers or lenses, a plurality of other optical devices augmented by an additional resin or the like, an additional coupling or a protective substance, or another optical device suitable for the purposes here.

As shown in Figure 2, light, indicated at 32, is emitted from the distal end of needle 18. In this embodiment, in use, the needle 18 is inserted into the joint of an animal or human, and light is emitted from optical fiber element or fiber optic 26 and transmitted through some thickness of biologic tissue in the area of the joint. After the light is backscattered by the biologic tissue into which needle 18 is inserted, the light is received back at the tip of needle 18 by optical fiber element or fiber optic 28.

Referring to Figure 5, a schematic cross-sectional view of handpiece 10 is shown which includes a block diagram of the handpiece 10 of Figure 1 with the needle assembly omitted. In this particular preferred embodiment, a friction coupling 34 is provided at the distal end of handpiece 10 which, in use, is coupled to the plastic mounting element or needle base 16 in which the base of needle 18 is received.

An optical assembly 36 is coupled to the fiber optical elements 26 and 28 of needle 18 and is connected to an opto-electronic converter circuit 38, preferably comprising a printed circuit board (PCB), which provides opto-electronic conversion of input and output signals and, in this regard, converts the optical signals received from optical assembly 38 into corresponding electrical (electronic) signals. Optical assembly 36 may comprise one or more lenses and/or other optical elements suitable for focusing, as needed, the transmitted and returned light.

Opto-electronic converter 38, which is of a conventional construction, is connected to a signal control and analysis circuit 40, also preferably comprising a PCB, which provides signal control and analysis of the electronic signals.

A readout and communication logic circuit 42, also preferably comprising a PCB, is connected to signal control analysis circuit 40. The functions of these circuits will be described in more detail below. It will be understood that the circuitry shown is merely exemplary and that, for example, different circuits can be combined in one unit on one PCB and, alternatively, more PCBs can be used.

It will be appreciated that handpiece 10 enables the user to guide and position needle 18 supported thereby and to control the depth of insertion of needle 18 into the joint.

Referring to Figure 6, a perspective view is shown of handpiece 10 coupled by a coupling cable 46 or the like to a separate control unit 44. Control unit 44 includes a chassis or housing 48 having an insignia 50 or other signage thereon and including a communications coupler 52, a bank 54 of switches or other controls, and an external readout or display 56. In some embodiments, some of the functions mentioned above or described below can be performed by control unit 44 rather than by the circuitry within handpiece 10.

Turning to Figure 7, a block diagram is shown of one preferred embodiment of the overall system illustrating the opto-electronic signal processing. An unmodulated power source 60, which, as indicated, may be part of circuit 40, drives a source light generation device or light generator 62 which, as indicated, may be part of circuit 38. In preferred embodiments, light generator 62 comprises a laser diode or a light emitting diode (LED).

Because the signal is intensity modulated by the design parameters of the laser diode, no exogenous modulation of light generator 62 is required. In one preferred implementation, the laser diode of light source or generator 62 is driven by a simple continuation voltage from power source 60 and the light generated is narrowband and continuous wave (CW). In this embodiment, the change in light intensity is the primary contrast medium. Light from light generator 62 is coupled through an optical coupling 64 of light coupling assembly 36 to fiber optic 26 (not shown in Figure 7) as described above. Optical coupling 64 preferably includes a gel-based impedance coupling. Alternatively, as indicated above, different couplings can be used.

Although light generator or light source 62 may comprise a laser diode or LED as mentioned above, light source 62 may also comprise, for example, another solid state device, a gas laser, a crystal laser, a filament lamp, a fluorescent lamp, or other light source. Further, although the light generated is narrow band and CW in one preferred embodiment, the light source 62 can be amplitude modulated, pulsed, frequency modulated, phase modulated, polarization modulated, monochromatic, multiple wavelength (producing light of different colors), broadband, or employ a further different modulation method or driving method.

In use, with needle 18 inserted into the joint of a patient (which may be a human or an animal), backscattered light from, e.g., biological tissue will be received by fiber optic 28 as described above and coupled through a further optical coupling 65 of optical coupling assembly 36 to an optical to electronic transduction module or circuit 6, which, as indicated, may be formed as part of circuit 38. In one preferred embodiment, optical coupling 65 also comprises a gel-based impedance coupling and circuit 66 comprises a conventional photodiode circuit. Alternatively, circuit or module 66 comprises a photomultiplier tube, or other optical device.

In one embodiment, the handpiece 10 and optical guide 25 are removed from needle 18 after needle placement, and the needle 18 is subsequently used for injection or aspiration after the attachment of a syringe. In an alternative embodiment, the aspiration or injection syringe function is built into handpiece 10, and thus removal of needle 18 from handpiece 10 is not required.

The output of circuit 66 is connected to an electronic preprocessing and filtering module or circuit 68 which is also connected to power source 60. In one preferred embodiment, as described in more detail below, the preprocessing employed comprises temporal averaging using signal latching.

The output of circuit 68 is connected to a parameter estimation module or circuit 70. Parameter estimator module 70 is used to determine the type of tissue in which the tip of needle 18 resides. In one preferred embodiment, described in more detail below, a look-up table is used in parameter estimation.

The parameter estimation module 70 is connected to readout logic circuit or readout module 42 mentioned above. As set forth in more detail below, in one preferred embodiment, readout module 42 displays the likelihood of the needle 18 being in the intra-articular space of, e.g., the knee into which needle 18 is injected, and, in particular, provides for activation of one of the three lights or lamps forming readout 22 depending on whether the likelihood is low, intermediate or high.

Referring to Figure 8, a further preferred embodiment of the invention is shown. This embodiment is similar to that of Figure 7 and like units have been given the same reference numerals. In this embodiment, an electronic modulation module or circuit 72 drives optical source 62 and an electronic demodulation module or circuit 74 demodulates the output of the optical to electrical transduction module 66 which may reference the electronic modulation. Optical coupling unit or modules 64 provides optical modulation while optical coupling unit or module 65 provides optical demodulation which may reference the optical modulation. Apart from these physical features, this embodiment is otherwise similar to that of Figure 7 apart from the differences in operation discussed below.

It is noted that in the general case of intensity modulation, the electronic signal is demodulated relative to the source electronic signal by comparison of the relative signal magnitude in order to determine the relative increase in intensity. In the case where a thermally stable laser source is used to implement light source 62, the demodulation is fed forward rather than held back. In one preferred embodiment, no specific calculation relative to the signal produced by optical source 62 needs to be undertaken by demodulation module 74. Electronic preprocessing module or circuit 68 provides preprocessing of the signal by temporal averaging to produce a single value (number) over a period which preferably is on the order of milliseconds to seconds. The magnitude of this single voltage, current or other parameter is preferably stored by a latch (not shown) in module 68. This process is described in more detail below.

In one preferred embodiment; the optical source 62 includes a second laser producing a light signal of a different wavelength. The process outlined above is repeated for the second laser to generate a second value (number) stored within a separate latch (now shown) in module 68. The magnitudes of the two latched values are used in their native form and converted to binary integers. These binary integers represent the magnitude of the intensity decrease for the two wavelengths of light produced by the two lasers or light source 62.

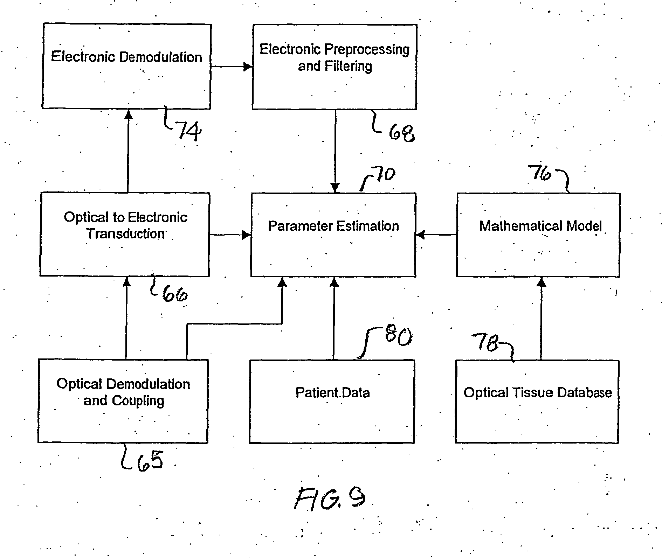

Turning to Figure 9, a block diagram is shown which depicts in more detail the parameter estimation aspect of the invention, in accordance with one embodiment thereof. Figure 9 includes, in addition, the output side of the embodiment of Figure 8. It will, of course, be understood that the principles discussed in connection with Figure 9 are applicable to the embodiment of Figure 7 and to other embodiments.

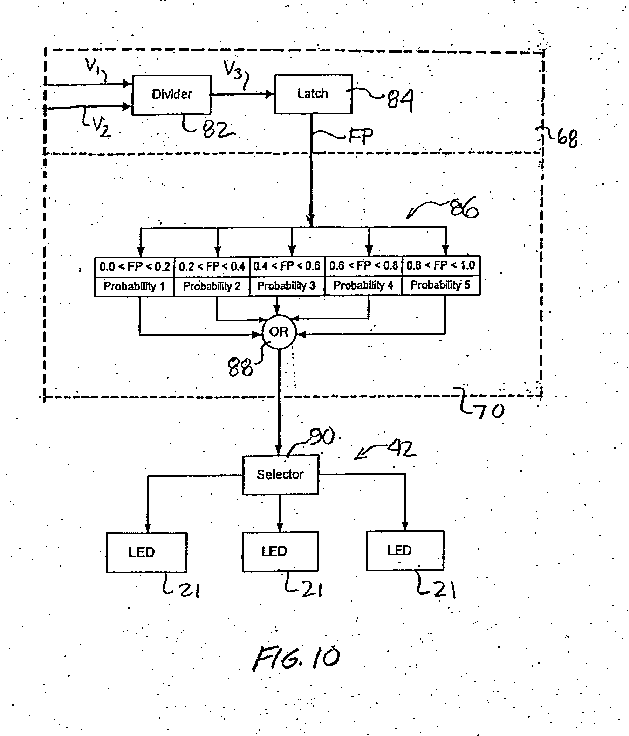

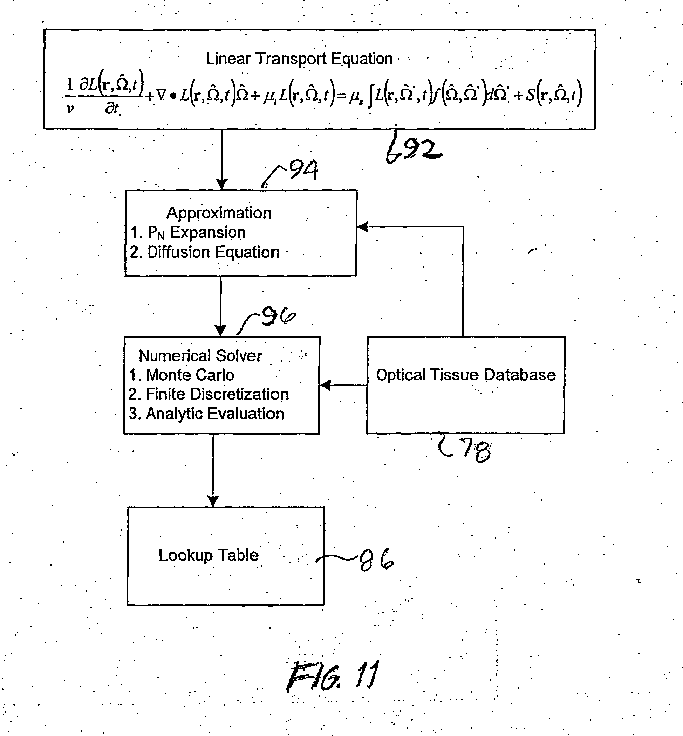

As indicated by block 76, a mathematical model of light propagation in biologic tissue is used in optimizing the parameter estimation represented by block 70. As indicated by block 78, this model is based on a database derived from repeated computer simulations of light propagation in biologic tissues. Further, as indicated by block 80, in addition to the computer simulated data, experimental patient data can also be used in parameter estimation. One preferred embodiment for performing parameter estimation is considered in more detail below in connection with Figures 10 and 11.

Referring to Figure 10, voltages V₁ and V₂ are, respectively, the unmodulated source voltage from unmodulated power source 60 of Figure 7 and the transduced voltage from the optical to electronic modulation module 66 of Figure 7. A voltage divider 82 provides an output voltage V₃, based on the ratio of voltages V₁ and V₂, which is averaged temporally over a predetermined time period (e.g., 100 milliseconds) and stored in a latch 84.

A lookup table 86 receives the floating point output of latch 84. In other words, the latched output of latch 84, which has a value between 0.0 and 1.0, is used as an input to lookup table 86. Lookup table 86, as illustrated, is implemented using cascaded logic so that, in this example, one of five probabilities is determined depending on the value of FP. The probabilities are ORed together using an OR gate 88 and the output of the latter is supplied to selector 90 which, depending on the output of OR gate 88, drives the three lights or lamps 21 described above, which, as shown, are each implemented by a light emitting diode (LED). In a specific, non-limiting implementation, a red light is used for low probability (e.g., 0.00-0.50), a yellow light for intermediate probability (e.g., 0.51-0.75) and a green light for high probability (e.g., 0.76-1.0). This readout is used by the human operator to iteratively reposition the tip of needle 18. The process of interrogating the needle tip position is repeated as needed until the position is satisfactory.

Turning to Figure 11, these is shown one preferred embodiment of the mathematical model 76 of Figure 9 which is used to populate a lookup table such as lookup table 86 of Figure 10. As illustrated by block 92, a linear transport equation is used which is based on a general theory of the propagation of optical photons in random-scattering medium, such as biologic tissue. Linear transport equations are discussed in, e.g., Case KM, Werfel PF., "Linear Transport Theory." Addison-Wesley. 1967. As described below, this theory can be simplified to so-called Pan approximations and diffusion equations under some circumstances and these are well described in the literature. As indicated by blocks 94 and 78, for tissue types in which scattering is approximately ten types greater than absorption, as determined by optical tissue database 78 (which corresponds to database 78 of Figure 9), analytical approximations to the linear transport equation of block 92 as used. As indicated, the approximation can be made based on Pn expansion or a diffusion equation, although other known methods of approximation can also be employed. Otherwise, the linear transport equation of block 92 is itself used.

Depending on the equation used, a numerical solver 96 uses one of three known methods, Monte Carlo, finite discretization or analytical evaluation to solve the equation in question. The solution to the equation provided by numerical solver 96 determines the input and output intensities which are the quantities needed to populate lookup table 86.

In summary, referring again to Figure 8, in one preferred embodiment, the binary numbers produced by module 68 are used as the input to lookup table of parameter estimation module 70 (and corresponding to lookup table 86 of Figure 10) that provides an estimate of the tissue type being sampled. This estimate takes the form of a probability, viz., a low, medium or high probability that the sample is being taken from the intra-articular space (synovial tissue or synovial fluid). It will be appreciated that while this is a preferred embodiment, in general, the injection target site may be any one of a joint, a muscle, a fascial layer or a fat layer.

As discussed above, in a preferred embodiment, the lookup table contains multiple parameters that must be estimated before its implementation in custom digital logic. In one implementation described above, Monte Carlo simulation of the propagation of photons in biological tissues of various scattering and absorption parameters is used to populate the lookup table of module 70. The lookup table also accounts for device characteristics (e.g., photodiode efficiency) and patient characteristics (e.g., scattering and adsorption of synovial tissue for experimental samples). The lookup table implements function approximation in an overall manner similar to the standard methods of parameter estimation (see, e.g., Haykin S., "Neural Networks: A Comprehensive Foundation," 2nd Edition. Prentice Hall. 1998).

It will be appreciated that the transmitted signal may be referenced in terms of intensity, amplitude, frequency, phase, polarization, or other parameters in an optical or electronic form. Further, signals in optoelectronic form may be processed with linear filters, matched filters, wavelet filters, time-domain filters, frequency domain filters, statistical time-series methods, or statistical filters.

In addition to the embodiments described above, parameters may be estimated by function approximation methods, function estimation methods, linear regression methods, nonlinear regression methods, neural networks, radial basis-function networks, fuzzy logic, or other multivariate function approximation or estimation methods. In addition, parameters may be estimated by solution of a forward model of differential equations, stochastic processes, or algebraic discretizations of these models or by means of matrix computation, analytic functions, or pseudo-random (Monte Carlo) methods. Further, the corresponding algorithms may be implemented with the use of optical components, electronic components, digital circuits, analog circuits, separate components integrated with the use of a printed circuit board, integrated circuits, application specific integrated circuits, programmable gate arrays, arithmetic logic units, microprocessors, firmware or software. It is also noted that the readout update may be real-time, periodic, or intermittent.

Accordingly, although the invention has been described above in relation to preferred embodiments thereof, it will be understood by those skilled in the art that variations and modifications can be effected in these preferred embodiments without departing from the scope and spirit of the invention.

## Claims

1. A device for assisting in positioning of a needle within a patient, said device comprising:
a handpiece for manipulation by a user;
a light source;
a needle assembly mounted on the handpiece, said needle assembly comprising a needle including a needle tip and a central lumen, and an optical guide, disposed in said lumen, for, in use with the needle inserted in the patient, transmitting light from said light source through said lumen so as to be emitted from the needle tip and receiving the light emitted from the needle tip that is returned to the needle tip from a location within the patient; and
processing means for processing the returned light to provide a determination of the location within the patient at which the needle tip is positioned.

2. A device as claimed in claim 1 wherein said optical guide comprises at least first and second fiber optics supported in said lumen.

3. A device as claimed in claim 1 wherein said processing means uses parameters related to both the transmitted and the returned light in providing said determination.

4. A device as claimed in claim 3 wherein said processing means compares the transmitted and returned light with respect to relative intensity.

5. A device as claimed in claim 4 wherein said processing means uses properties of different body substances in said determination.

6. A device as claimed in claim 5 wherein said determination of said processing means includes discriminating between body substances selected from the group consisting of connective tissue, muscle, fat, synovial tissue, synovial fluid, and intra-articular connective tissue.

7. A device as claimed in claim 1 further comprising readout means for displaying an indication of the probability that the needle tip is positioned in an intra-articular space within the patient.

8. A device as claimed in claim 1 further comprising a parameter estimation module, and a power supply for producing an electrical output for powering the light source, and wherein the processing means includes means for converting the returned light into an electrical signal, and means for comparing the electrical signal and said electrical output from said power supply for the light source to provide an input to said parameter estimation module.

9. A device as claimed in claim 1 wherein at least part of said processing by said processing means takes place within the handpiece.

10. A device for assisting in positioning of a needle within a patient, said device comprising:
a handpiece for manipulation by a user;
a light source;
a needle assembly mounted on the handpiece, said needle assembly comprising a needle including a needle tip and a central lumen and an optical guide disposed in said lumen and including at least a first optical fiber for, in use with the needle inserted in the patient, transmitting light from said light source through said lumen so as to be emitted from the needle tip and at least a second optical fiber for, in use with the needle inserted into the patient, receiving the light emitted from the needle tip that is returned to the needle tip from a location within the patient;
processing means for processing the returned light to provide a determination of the location within the patient at which the needle tip is positioned; and
a readout, connected to said processing means, for indicating to a user, based on said determination, a probability of the needle tip being located at a predetermined location in the patient.

11. A device as claimed in claim 10 wherein said readout comprises at least two different light outputs indicating at least two different probabilities that the needle tip is located at said predetermined location.

12. A device as claimed in claim 11 wherein said predetermined location is within an intra-articular space within the patient.

13. A method for positioning a needle within a patient, said method comprising:
providing a device including a needle including a needle tip and a lumen, and an optical guide disposed in the lumen of the needle and extending to the needle tip;
positioning the needle within a body substance of a patient by piercing the skin and soft tissue of the patient;
causing light to be transmitted through the optical guide so as to be emitted from the needle tip into the patient;
using the optical guide to receive light returned from the body substance of the patient in which the needle tip is positioned; and
processing the returned light to provide a determination of the body substance in which the needle tip is positioned.

14. A method as claimed in claim 12 wherein parameters relating to both the transmitted light and the returned light are processed in providing said determination.

15. A method as claimed in claim 12 wherein the transmitted and returned light are compared with respect to relative intensity.

16. A method as claimed in claim 15 wherein properties of different body substances are used for said determination.

17. A method as claimed in claim16wherein said determination includes discriminating between body substances selected from the group consisting of connective tissue, muscle, fat, synovial tissue, synovial fluid, and intra-articular connective tissue.

18. A method as claimed in claim 13 further comprising displaying an indication of the probability that the needle tip is positioned in an intra-articular space within the patient.

19. A method as claimed in claim18further comprising repositioning the needle, as needed, until the indication displayed represents an acceptable probability that the needle tip is positioned in the intra-articular space.

20. A method as claimed in claim 13 wherein the transmitted light is produced by a light source, wherein the returned light is converted into an electrical signal, and wherein the electrical signal and an electrical signal from a power supply for the light source are processed to provide an input in a parameter estimation process that provides said determination.

21. A method as claimed in claim 13 wherein the device comprises a handpiece and at least part of said processing takes place within the handpiece.
